# EUROPEAN PATENT APPLICATION

(11) **EP 4 559 314 A1**
(43) Date of publication of application: **28.05.2025**
(21) Application number: 23307039.0
(22) Date of filing: 23.11.2023
(51) Int. Cl.: A01P 1/00, A01N 59/16, A01N 25/28, A01N 25/34, A01N 43/90

(54) **A MAGNETIC ANISOTROPIC MATERIAL FOR DISRUPTION AND/OR SENSITIZATION OF BACTERIAL BIOLFILM**

(71) Applicant: Centre National de la Recherche Scientifique, 75016 Paris (FR); UNIVERSITE TOULOUSE III - PAUL SABATIER, 31062 Toulouse Cedex 9 (FR); Jozef Stefan Institute, 1000 Ljubljana (SI)
(72) Inventor: KOLOSNJAJ-TABI, Jelena, 31077 Toulouse Cedex 04 (FR); NEMEC, Sebastjan, 6271 Dekani (SI); GOLZIO, Muriel, 31077 Toulouse Cedex 04 (FR); ROLS, Marie-Pierre, 31077 Toulouse Cedex 04 (FR); DA SILVA, Charlotte, 31077 Toulouse Cedex 04 (FR); CAF, Maja, 1000 Ljubljana (SI); MERTELJ, Alenka, 1000 Ljubljana (SI); MAKOVEC, Darko, 1000 Ljubljana (SI); KRALJ, Slavko, 1000 Ljubljana (SI)
(74) Representative: IPAZ

(57) **Abstract**

The present invention relates to a magnetic anisotropic material, in the form of magnetic elongated elements, preferably superparamagnetic nanochains, for use in the disruption of a bacterial biofilm and/or in the sensitization of bacterial colonies to antibiotic treatment, the non-therapeutical use of said magnetic anisotropic material for disrupting a bacterial biofilm and/or sensitizing bacterial colonies present on a surface, on and/or within a medical device, in pipes, in conducts, and/or in a liquid, a biofilm disrupting and/or sensitizing composition comprising said magnetic anisotropic material and at least one antibacterial agent, said biofilm disrupting and/or sensitizing composition for use in the treatment of a bacterial infection or infestation involving biofilm formation, the non-therapeutical use of said composition as an anti-infective agent, preferably for the disinfection of a surface, a medical device, pipes, and/or conducts, or for purification of a liquid, and an *in vitro* or *ex vivo* method for disrupting a bacterial biofilm and/or sensitizing bacterial colonies to antibiotic treatment, wherein it comprises a step of applying an oscillating or rotating magnetic field to a magnetic anisotropic material.

## Description

The present invention relates to a magnetic anisotropic material, in the form of magnetic elongated elements, preferably superparamagnetic nanochains, for use in the disruption of a bacterial biofilm and/or in the sensitization of bacterial colonies to antibiotic treatment, the non-therapeutical use of said magnetic anisotropic material for disrupting a bacterial biofilm and/or sensitizing bacterial colonies present on a surface, on and/or within a medical device, in pipes, in conducts, and/or in a liquid, a biofilm disrupting and/or sensitizing composition comprising said magnetic anisotropic material and at least one antibacterial agent, said biofilm disrupting and/or sensitizing composition for use in the treatment of a bacterial infection or infestation involving biofilm formation, the non-therapeutical use of said composition as an anti-infective agent, preferably for the disinfection of a surface, a medical device, pipes, and/or conducts, or for purification of a liquid, and an *in vitro* or *ex vivo* method for disrupting a bacterial biofilm and/or sensitizing bacterial colonies to antibiotic treatment, wherein said method comprises a step of applying an oscillating or rotating magnetic field to a magnetic anisotropic material.

Antibiotic resistance has been gaining ground and is currently, according to the WHO, among the greatest threats to global health. The therapeutic efficiency of antibiotics may decrease over time, as bacteria acquire antimicrobial resistance. The resistance can be due to different mechanisms, such as the occurrence of drug efflux and decreased uptake of antibiotic agents, modification of targets on the surface of bacteria, and release of agents that inactivate drugs or prevent their penetration into bacterial surroundings. One of the main mechanisms involves bacterial secretion of biofilm: a slime layer that hampers the diffusion of molecules and acts as a protective shield. This bacterial extracellular matrix represents a particularly challenging therapeutic barrier. The bacterial biofilm is composed of homogenous or heterogeneous bacterial communities, which secrete a thick matrix containing extracellular polymeric substances (EPSs), like extracellular polysaccharides, proteins, lipids, DNA and humic substances. This bacterial extracellular matrix allows the cells to stick between themselves and to adhere to the surface, as well as it acts as a protective barrier that shields bacteria from external threats, such as the host's immune response, mechanical and shear forces, nutrients scarcity, external pH, or antimicrobial agents. As a concrete example, methicillin resistance is notably widespread and is characteristic for aggregated communities of bacteria, such as the ones that generate biofilms. Biofilms are among the major causes for failure of antibiotic treatments and account for numerous chronic infections.

In order to disrupt this complex matrix, antibacterial treatments often require physical procedures, like local scraping of infected surfaces. However, such procedures are not always possible and are generally limited to (removable) medical devices disinfection procedures. Alternatively, the treatments require the administration of large doses of antibiotic agents to hosts, which in turn may lead to severe adverse reactions within treated patients, or, the infections simply cannot be treated by antibiotic agents.

Magnetic particles were described to improve the efficacy of antibiotic agents. For example, the patent application CN109908172A describes a magnetic nanocomposite comprising elemental selenium nanoparticles and magnetic iron oxide nanoparticles. Under the action of an external magnetic field, the magnetic iron oxide nanoparticles can infiltrate the selenium nanoparticles of the nanocomposite into the bacterial biofilm, thereby significantly enhancing the treatment effect on the biofilm (synergistic effect of selenium and iron oxide). This approach nevertheless implements selenium which may have toxic effects. Additionally, there is no mention of nanocomposite amounts required to provide an antibacterial effect and according to CN109908172A, the treatment is not completely satisfactory since live cells are still present.

The research on magnetic nanoparticles performed until today mainly focused on spherical magnetic nanoparticles potential to be attracted by a static magnetic field and thus build channels in bacterial matrix, but biofilms were not disintegrated by such protocols.

Consequently, there is still a need to provide new broadly acting tools to efficiently disrupt bacterial biofilms and/or sensitize bacterial colonies to antibiotic treatment, which are easy to fabricate as well as to implement, which can be produced at large scale, and/or which can be used in entangled compartments and other hard-to-reach locations.

A first object of the present invention is a magnetic anisotropic material in the form of magnetic elongated elements, each elongated element comprising core(s) of metal and/or metal oxide particle(s), for use in the disruption of a bacterial biofilm and/or in the sensitization of bacterial colonies to antibiotic treatment.

Thanks to the anisotropic shape of the magnetic material (elongated elements), it is possible to remotely trigger an oscillatory or rotational movement of the magnetic anisotropic material by applying an oscillating or rotating magnetic field, which can, together with the magnetic field gradient, multidirectionally move elongated elements and result in targeted multidirectional movements, which efficiently disrupt the bacterial biofilm and/or sensitize bacterial colonies to antibiotic treatment. The magnetic anisotropic material can be actuated not only by magnetic field gradients but also by non-static magnetic field such as an oscillating or rotating magnetic field. This allows for magnetic unidirectional movement as well as magnetic rotational movement or oscillation which favorably disrupts the bacterial biofilm and/or sensitize bacterial colonies to antibiotic treatment. The magnetic anisotropic material as defined in the present invention thus locally exerts physical actions, such as induces magneto-mechanical forces, which can loosen and/or disrupt the biofilm upon the application of an oscillating or rotating magnetic field.

In particular, the anisotropic shape of the material or elements comprised in said material has an important advantage compared to spherical elements or materials, because anisotropic elements or materials can rotate or oscillate when they are exposed to a preferably oscillating or rotating magnetic field at low frequencies and low intensities. This is an important feature, which allows exploiting multi-directional magneto-mechanical effects on soft matter.

The magnetic anisotropic material is biocompatible and non-toxic to cells.

The magnetic anisotropic material is in the form of magnetic elongated elements, and preferably in the form of a suspension comprising several or multiple magnetic elongated elements.

The elongated elements have preferably a shape factor of at least about 1.5, more preferably ranging from about 3 to about 15, and even more preferably from about 5 to about 10.

More particularly, the elongated elements can be defined as elements having:
- a length (L1), extending in a main direction of elongation,
- two dimensions (D1) and (D2), referred to as orthogonal dimensions, extending along two transverse directions that are orthogonal to one another and orthogonal to said main direction of elongation, said orthogonal dimensions (D1, D2) being smaller than said length (L1) and less than 5 µm, and,
- two ratios (F1) and (F2), referred to as shape factors, between said length (L1) and each of the two orthogonal dimensions (D1) and (D2) respectively, said shape factors (F1, F2) being at least about 1.5, more preferably ranging from about 3 to about 15, and even more preferably from about 5 to about 10.

The expression "shape factor" means the ratio between the length (L1) of an elongated element, and one of the two orthogonal dimensions (D1, D2) of said elongated element.

According to one preferred embodiment, the two orthogonal dimensions (D1, D2) of an elongated element are the diameter (D) of its transverse cross section. It is then referred to as a "rod" or "wire" or "chain" or "tape" or "bundle" or "ribbon".

The elongated element may also be a "platelet" in which the two orthogonal directions of the elongated element are its width (W) (first orthogonal dimension) and its thickness (E) (second orthogonal dimension).

More particularly, the elongated elements are advantageously characterized by at least one of the following features:
- the two orthogonal dimensions (D1, D2) of elongated elements range from 20 nm to 400 nm approximately, preferably from 60 nm to 300 nm approximately, and more preferably from 90 nm to 150 nm approximately;
- the length (L1) ranges from 50 nm to 1 mm approximately, preferably from 200 nm to 30 µm approximately, and even more preferably from 300 nm to 2 µm approximately.

The anisotropic magnetic material has preferably a coercive field ranging from 0 Oe to 2000 Oe approximately, and more preferably ranging from 0 Oe to 300 Oe approximately. When the coercive field is zero, it is a superparamagnetic material. In approximation the particle with the highest coercivity defines the coercivity of the whole material.

The anisotropic magnetic material is preferably characterized by magnetization measured at the field strength applied during their use in the disruption and/or in the sensitization of a bacterial biofilm ranging from 2 emu/g to 100 emu/g approximately, and more preferably from 6 emu/g to 30 emu/g, approximately.

In one preferred embodiment, the elongated elements are superparamagnetic elongated elements, and more preferably superparamagnetic nanochains.

Elongated elements or nanochains are referred to as "superparamagnetic" because the curve of their magnetization as a function of an applied magnetic field resembles that of a paramagnetic material since they show no coercive magnetic field and remanent magnetization at room temperature, while superparamagnetic materials, in contrast to paramagnetic material, express high magnetic susceptibility. Superparamagnetic elongated elements have the double advantage of being strongly attracted by a magnet due to their relatively large size, high magnetic susceptibility and high saturation magnetization, and of not magnetically aggregating in the absence of an applied magnetic field (i.e. absence of spontaneous magnetization). The magnetization of such elongated elements under the effect of an applied magnetic field no longer describes a magnetic hysteresis loop. Elongated elements of such a material can then be dispersed in a liquid to form a stable suspension because there are no magnetic attractive dipole interactions among elongated elements when the suspension is removed from the magnetic field source.

Each elongated element comprises core(s) of metal and/or metal oxide particle(s).

Each core is composed of or comprises one or more metal and/or metal oxide particles, and preferably is composed of or comprises several metals and/or metal oxide particles.

In one preferred embodiment, the cores of metal and/or metal oxide particle(s) are clusters of nanoparticles.

The cores or clusters can comprise or can be composed of from about 1 to about 300 particles or nanoparticles.

The cores or clusters can have a size ranging from 20 nm to 700 nm approximately, and more preferably ranging from 70 nm to 200 nm approximately.

The metal and/or metal oxide particles are composed of or comprise a metal, a metal oxide or a mixture of a metal and a metal oxide.

In one preferred embodiment, each elongated element comprises several cores of metal and/or metal oxide particle(s), at least two cores of metal and/or metal oxide particle(s), and more preferably from 3 to 15 cores of metal and/or metal oxide particle(s).

The metal and/or metal oxide particles can be selected from particles, and preferably nanoparticles, comprising at least one metal selected from Fe, Co, and Ni.

Iron-based particles, and advantageously iron-based nanoparticles, are preferred.

In one particularly preferred embodiment, the metal and/or metal oxide particles are iron oxide nanoparticles.

The metal and/or metal oxide particles can have a size ranging from 3 nm to 500 nm approximately, and more preferably ranging from 5 nm to 70 nm approximately.

When the particles are nanoparticles, they have a size as defined above but of at most 100 nm approximately.

In one preferred embodiment, each elongated element (forming the magnetic anisotropic material) is encapsulated in an inorganic or organic material shell or is randomly embedded in an inorganic or organic material matrix. The presence of the inorganic or organic material as a shell or as a matrix enables mechanical and structural rigidity of the elongated element.

In one preferred embodiment, the cores of metal and/or metal oxide particle(s) are aligned so as to form said each elongated element. It enables the fixation of the shape of the elongated element by the inorganic or organic material.

Preferably, the cores are fixated into said inorganic or organic material shell or matrix.

This embodiment is particularly advantageous when each elongated element is encapsulated in an inorganic or organic material shell. The aligned cores are advantageously encapsulated in said inorganic or organic material shell. Preferably, the cores are centred into said inorganic or organic material shell.

In one preferred embodiment, the shell can have a thickness ranging from 1 nm to 80 nm approximately.

The inorganic or organic material (of the shell or of the matrix) can be selected from metal oxides, polymers, carbon, and silicon, and preferably from metal oxides.

Examples of polymers can include polystyrene, polymethylmethacrylate, polydimethylsiloxane, polydopamine, polylactic-co-glycolic acid, dextran, chitosan, alginate, and mixtures thereof.

Examples of metal oxides can include TiOz, Al₂O₃, ZnO, or SiOz, and preferably SiOz.

Examples of carbon can include amorphous carbon, graphite, graphene or doped carbon.

The organic or inorganic material can be a porous organic or inorganic material. It enables a delivery of cargo (macro)molecules or particles and increases effective surface of elongated element.

In one preferred embodiment, the shell (respectively the matrix) is an inorganic shell (respectively is an inorganic matrix).

Advantageously, the shell (respectively the matrix) is an inorganic porous shell (respectively is an inorganic porous matrix).

The elongated elements can be positively or negatively charged, and preferably negatively charged.

Indeed, the magnetic anisotropic material can be functionalized by appropriate chemical groups or moieties so as to be positively or negatively charged. When the magnetic anisotropic material is positively or negatively charged, binding of the material to different sites of biofilm aggregates can be promoted. Surface charge is monitored by measuring Zeta potential of the magnetic anisotropic material and can improve binding to bacterial aggregates.

Examples of chemical groups which lead to positively charged magnetic anisotropic material can include amine functional group(s), quaternary ammonium functional group(s), sulfonium functional group(s), and phosphonium functional group(s).

Examples of chemical groups which lead to negatively charged magnetic anisotropic material can include carboxylate functional group(s), silanol OH functional group(s), phosphate functional group(s), and sulphate functional group(s).

Preferably, the magnetic anisotropic material has a Zeta potential ranging from 0 mV to - 40 mV approximately.

In the present invention, the magnetic anisotropic material leads to disruption of a bacterial biofilm and/or sensitization of bacterial colonies to antibiotic treatment.

In the present invention, the term "disruption" means that the magnetic anisotropic material leads to at least reduction, and preferably elimination of the bacterial biofilm (i.e. at least partial removal, and preferably complete removal of the bacterial biofilm).

In the present invention, the term "sensitization of bacterial colonies to antibiotic treatment" means that addition/presence of said magnetic anisotropic material sensitises bacterial colonies to an antibiotic treatment, i.e. improves antibacterial activity of said bacterial colonies to said antibiotic treatment.

In particular, the magnetic anisotropic material according to the present invention is a material devoid of antibacterial activity and has the ability to potentiate and improve the effect of an antibiotic (or antibacterial agent) against a bacterial strain, and more particularly against a resistant bacterial strain. In short, one can consider that once co-administered with an antibiotic (or antibacterial agent), said magnetic anisotropic material "suppresses" the resistance and "improves" the inhibitory effect of an antibiotic.

Thus, the magnetic anisotropic material is able to restore or enhance the antibacterial activity of antibiotics, and more particularly to restore or enhance the antibacterial activity of existing antibiotics in bacteria that form the biofilm. The magnetic anisotropic material according to the present invention is therefore useful for the treatment of Gram-positive and Gram-negative bacteria such as resistant Gram-positive and resistant Gram-negative bacterial strains, while guaranteeing low toxicity towards humans and animals.

"Gram-negative bacteria" refers to bacteria that do not retain the crystal violet stain used in the Gram staining. Gram-negative bacteria are characterized by a bacterial cell wall composed of a thin layer of peptidoglycan in between an inner cytoplasmic cell membrane and a bacterial outer membrane.

The Gram-negative bacteria are preferably selected from *Pseudomonas* bacteria, *Escherichia* bacteria, *Klebsiella* bacteria, *Acinetobacter* bacteria, *Enterobacter* bacteria and *Legionella* bacteria.

Examples of *Pseudomonas* bacteria are *Pseudomonas aeruginosa.*

Examples of *Escherichia* bacteria are *Escherichia coli.*

Examples of *Klebsiella* bacteria are *Klebsiella pneumoniae.*

Examples of *Acinetobacter* bacteria are *Acinetobacter baumannii.*

"Gram-positive bacteria" refers to bacteria that retain the crystal violet stain used in the Gram staining. Gram-positive bacteria are characterized by a thick layer of peptidoglycan as a bacterial outer layer.

The Gram-positive bacteria are preferably selected from *Staphylococcus* bacteria.

Examples of *Staphylococcus* bacteria are *Staphylococcus epidermidis, Staphylococcus aureus, Staphylococcus intermedius or Staphylococcus faecalis.*

The antibacterial agent or antibiotic is preferably selected from β-lactams, aminoglycosides, macrolides, fluoroquinolones, tetracyclines and sulphonamides, and more preferably selected from β-lactams.

In one particularly preferred embodiment, the magnetic anisotropic material is in the form of superparamagnetic nanochains, each nanochain comprising iron oxide nanoparticles clusters, each nanochain being encapsulated in a porous silica shell. The iron oxide nanoparticles clusters are assembled into a one-dimensional chain-like structure. The resulting magnetic anisotropic material is magneto responsive.

A second object of the present invention is the non-therapeutical use of a magnetic anisotropic material as defined in the first object of the present invention, for disrupting a bacterial biofilm and/or sensitizing bacterial colonies present on a surface, on and/or a medical device (e.g. implants, catheters, gastrointestinal ducts), in pipes (fine and/or entangled pipes), in conducts, and/or in a liquid.

Indeed, the magnetic anisotropic material is also useful for disrupting a bacterial biofilm and/or sensitizing bacterial colonies present on a surface, on and/or within a medical device (e.g. on a surface of orthopaedic implants, such as artificial hips, and/or within the lumen of catheters), in pipes, ducts and/or in a liquid. More particularly, it can be used in medical devices such as implants, catheters or gastrointestinal ducts, and in fine and/or entangled pipes, where conventional physical or chemical means cannot be used efficiently at least because they are not configured to reach places/areas that are known to be difficult to access.

In this second object, the magnetic anisotropic material is not used or applied to a human or an animal. As a result, the magnetic anisotropic material is applied to an object such as a medical device, for example a protheses, or to a surface of said object for disinfection of such object or surface.

The magnetic anisotropic material can be mixed with a liquid or a fluid for purification of such liquid or fluid.

In this second object, the magnetic anisotropic material as defined in the present invention is used as an aid improving the effect of an antibacterial agent.

A third object of the present invention is a biofilm disrupting and/or sensitizing composition comprising a magnetic anisotropic material as defined in the first object of the present invention and at least one antibacterial agent.

The antibacterial agent and the magnetic anisotropic material are administered in amounts that together are more effective to inhibit bacteria than administration of the same amount of the antibacterial drug without the magnetic anisotropic material.

The antibacterial agent or antibiotic is preferably selected from β-lactams, aminoglycosides, macrolides, fluoroquinolones, tetracyclines and sulphonamides, and more preferably selected from β-lactams.

Examples of β-lactams can include penicillins, cephalosporins, monobactams, and carbapenems.

Examples of macrolides can include erythromycin, clarithromycin, azithromycin, josamycin, roxithromycin, spiramycin, dirithromycin, or tylosin.

Examples of tetracyclines are tetracycline, doxycycline, minocycline, or tigecycline.

The magnetic anisotropic material can be comprised in the biofilm disrupting and/or sensitizing composition in an amount such that the metal and/or the metal oxide of said magnetic anisotropic material has a molar metal concentration in said composition ranging from about 0.01 mM to about 100 mM, and preferably ranging from about 0.05 mM to about 10 mM.

The biofilm disrupting and/or sensitizing composition preferably comprises a therapeutically effective amount of the antibacterial agent.

"Therapeutically effective amount" (in short "effective amount") refers to the amount of a therapeutic agent that is sufficient to achieve in the presence of the magnetic anisotropic material the desired therapeutic, prophylactic or preventative effect in the subject in need thereof to which/whom it is administered, without causing significant negative or adverse events or reactions (also referred to as side effects) to said patient. A therapeutically effective amount may be administered prior to the onset of the disease, disorder, or condition, for a prophylactic or preventive action. Alternatively, or additionally, the therapeutically effective amount may be administered after initiation of the disease, disorder, or condition, for a therapeutic action.

Preferably, the antibacterial agent is administrated at a concentration Caa (in µM), where Caa ≤ MICaa/2, and MICaa represents the minimum inhibitory concentration (in µM) of said antibacterial agent with respect to a given bacterial strain.

A potentiator or restoration effect is observed when a MIC ratio is greater than 1, where the MIC ratio = MIC of said antibacterial agent alone for said strain (in µM) / MIC of said antibacterial drug in the presence of said magnetic anisotropic material (in µM).

The biofilm disrupting and/or sensitizing composition is preferably an aqueous composition, i.e. it comprises water as a solvent.

Alternatively, the solvent can be an alcohol, such as ethanol.

The biofilm disrupting and/or sensitizing composition may contain inorganic or organic solutes, i.e. sodium chloride, or other physiologically relevant salts; bacteriostatic agents such as benzyl alcohol; lactate or dextrose, etc.

The biofilm disrupting and/or sensitizing composition may further comprise a pharmaceutically acceptable carrier.

"Pharmaceutically acceptable" means that the ingredients of the biofilm disrupting and/or sensitizing composition are compatible with each other and not deleterious to the patient to which/whom it is administered.

"Pharmaceutically acceptable carrier" refers to an excipient that does not produce an adverse, allergic or other untoward reaction when administered to an animal or a human. It includes any and all solvents, dispersion media, coatings, antifungal agents, isotonic and absorption delaying agents and the like. For human administration, preparations should meet sterility, pyrogenicity, general safety and purity standards as required by regulatory offices, such as, e.g., FDA Office or EMA.

Examples of pharmaceutically acceptable carriers include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulphate, disodium hydrogen phosphate, potassium hydrogen phosphate, sodium chloride, zinc salts, colloidal silica, magnesium trisilicate, polyvinyl pyrrolidone, dextran, cellulose-based substances (for example sodium carboxymethylcellulose), polyethylene glycol, polyacrylates, waxes, polyethylene- polyoxypropylene- block polymers, polyethylene glycol and wool fat.

A fourth object of the present invention is a biofilm disrupting and/or sensitizing composition as defined in the third object of the invention, for use in the treatment of a bacterial infection or infestation involving biofilm formation.

The combination of said magnetic anisotropic material and an antibacterial agent can be used for the treatment of a bacterial infection or infestation involving biofilm formation.

The bacterial infection or infestation involving biofilm formation can be selected from a chronic infection, a relapsing infection, a recalcitrant infection, or a persistent infection, including wounds and injuries.

A fifth object of the present invention is the non-therapeutic use of a biofilm disrupting and/or sensitizing composition as defined in the third object of the invention, as an anti-infective agent, preferably for the disinfection of a surface, a medical device (e.g. implants, catheters, gastrointestinal ducts), pipes (fine and/or entangled pipes), and/or conducts, or for the purification of a liquid.

A sixth object of the present invention is an *in vitro* or *ex vivo* method for disrupting a bacterial biofilm and/or sensitizing bacterial colonies to antibiotic treatment, wherein it comprises at least the following steps:
i) contacting a magnetic anisotropic material and an antibacterial agent with a bacterial biofilm or bacterial colonies, and
ii) applying a non-stationary magnetic field, and preferably an oscillating or rotating magnetic field.

Thanks to the method of the present invention and in particular the use of a magnetic anisotropic material, it is possible to remarkably enhance the antibacterial activity of antibacterial drugs against the resistant bacteria, and thus loosen or disrupt a bacterial biofilm and/or sensitize bacterial colonies to antibiotic treatment.

The magnetic anisotropic material combines magnetic force and torque-driven movement to affect bacterial aggregates and protrude throughout the bacterial biofilm. These interactions, in combination with an antibiotic drug, loosen or destroy the bacterial extracellular matrix and can be used as adjuvant strategy for biofilm eradication.

The antibacterial agent is as defined in the third object of the present invention.

The magnetic anisotropic material is preferably as defined in the first object of the present invention.

Due to a magnetic core, anisotropic shape, and electrostatic charge, the magnetic anisotropic material as defined in the first object of the present invention combines magnetic force and torque-driven movement and electrostatic interactions to attach to bacterial aggregates and protrude throughout the bacterial biofilm. These interactions, in combination with an antibiotic drug, loosen or destroy the bacterial extracellular matrix and can be used as adjuvant strategy for biofilm eradication.

In one preferred embodiment, step i) is implemented by contacting a suspension comprising from 0.05 to 20 mM of metal of said magnetic anisotropic material.

In one preferred embodiment, the antibacterial agent is used in a concentration Caa (in µM), where Caa ≤ MICaa/2, and MICaa represents the minimum inhibitory concentration (in µM) of said antibacterial agent with respect to a given bacterial strain.

MICaa is for example obtained by contacting said antibacterial agent alone (i.e. without the presence of said magnetic anisotropic material) with said bacterial biofilm or said bacterial colonies.

In one particularly preferred embodiment, step i) is implemented with a biofilm disrupting and/or sensitizing composition as defined in the third object of the present invention.

Before step ii) (i.e. in the absence of an oscillating or rotating magnetic field or any magnetic field), the magnetic elongated elements are randomly oriented.

The non-stationary magnetic field of step ii) can be uniaxial alternating, vibrating, pulsed, chaotic, rotating, or oscillating, and preferably rotating, or oscillating.

Step ii) is preferably carried out at low frequencies, i.e. at a frequency of at most 2000 Hz, and more preferably at a frequency ranging from about 0.1Hz to about 200 Hz.

Step ii) is preferably carried out at low magnetic field strength, i.e. at magnetic field strength of at most 1 T, and more preferably at magnetic field strength ranging from about 0.1 mT to about 120 mT.

During step ii), several elongated elements can assemble *in situ* to form transient microbundles which can then rotate or oscillate, involving bacterial biofilm disruption and/or bacterial colonies sensitization to antibiotic treatment.

Step ii) can be carried during from about 10 min to about 72h, and preferably during from about 6h to about 48h.

The highly magneto-responsive (anisotropic) (superpara)magnetic material, which can be activated by a low frequency and low magnetic field strengths, is thus used in step ii) as microscopic magnetic stirring bars or nanoscopic beaters, to locally loosen or destructure the bacterial biofilm, enhance the penetration, and thus improve the effect of antibiotic agents. This method therefore allows to treat the biofilm-related infections in acute and chronic skin wounds, as well as to treat biofilm infections related to implanted medical devices (such as catheters and prosthetic implants).

The method preferably does not comprise a step of irradiation, such as laser or IR or UV irradiation.

The invention also relates to a kit comprising:
- a pharmaceutical composition comprising a magnetic anisotropic material as defined in the first object of the present invention, and
- a separate pharmaceutical composition comprising at least one antibacterial agent.

The antibacterial agent is defined in the third object of the present invention.

The present invention further relates to a method for treating a bacterial infection or infestation involving biofilm formation in a subject in need thereof, comprising administering to the subject a magnetic anisotropic material and an antibacterial agent as defined in the sixth object of the present invention or a biofilm disrupting and/or sensitizing composition as defined in the third object of the present invention, and applying a non-stationary magnetic field, and preferably an oscillating or rotating magnetic field.

The present invention further relates to a method for treating a chronic infection, a relapsing infection, a recalcitrant infection, or a persistent infection, including wounds and injuries, in a subject in need thereof, comprising administering to the subject a magnetic anisotropic material and an antibacterial agent as defined in the sixth object of the present invention or a biofilm disrupting and/or sensitizing composition as defined in the third object of the present invention, and applying a non-stationary magnetic field, and preferably an oscillating or rotating magnetic field.

The present invention further relates to the use of a magnetic anisotropic material as defined in the first object of the present invention or a biofilm disrupting and/or sensitizing composition as defined in the third object of the present invention, for the manufacture of a medicament for the treatment of a bacterial infection or infestation involving biofilm formation in a subject in need thereof.

The present invention further relates to the use of a magnetic anisotropic material as defined in the first object of the present invention or a biofilm disrupting and/or sensitizing composition as defined in the third object of the present invention, as described herein, for the manufacture of a medicament for the treatment of a chronic infection, a relapsing infection, a recalcitrant infection, or a persistent infection, including wounds and injuries, in a subject in need thereof.

The present invention also relates to the use of a magnetic anisotropic material as defined in the first object of the present invention or a biofilm disrupting and/or sensitizing composition as defined in the third object of the present invention, for treating a bacterial infection or infestation involving biofilm formation in a subject in need thereof.

The present invention also relates to the use of a magnetic anisotropic material as defined in the first object of the present invention or a biofilm disrupting and/or sensitizing composition as defined in the third object of the present invention, for treating a chronic infection, a relapsing infection, a recalcitrant infection, or a persistent infection, including wounds and injuries, in a subject in need thereof.

"Bacterial infection" or "bacterial infestation" refers to any undesired presence and/or growth of bacteria as pathogen in a subject. Such undesired presence of microorganism may have a negative effect on the host subject's health and well-being. While the term "bacterial infection" or "bacterial infestation" should not be taken as encompassing the normal growth and/or presence of microorganism which are normally present in the subject, for example in the digestive tract of the subject, it may encompass the pathological overgrowth of such microorganism. "Bacterial infection" or "bacterial infestation" is a pathologic condition or disorder caused by the growth and/or presence of bacteria as microorganism. Therapeutic agents for the treatment of "bacterial infection" or "bacterial infestation" are "antibacterial" agents or drugs.

"Chronic infection", "relapsing infection", "recalcitrant infection" and "persistent infection" refer to bacterial infection which resists to the host immune system and antibiotic treatments and is capable of reactivation into clinically significant disease with chronic symptoms.

In one embodiment, the bacterial infection is selected from cystic fibrosis, urinary tract infection, chronic otitis, and pneumonia.

In one preferred embodiment, the bacterial disease is caused by Gram-positive bacteria.

"Subject" refers to an animal, typically a warm-blooded animal, preferably a mammal. The term "mammal" refers here to any mammal, including humans, domestic and farm animals, and zoo, sports, or pet animals, such as dogs, cats, cattle, horses, sheep, pigs, goats, rabbits, etc. Preferably, the mammal is a primate, more preferably a human.

"Human" refers to a male or female human subject at any stage of development, including neonate, infant, juvenile, adolescent, adult, and elderly.

In one embodiment, the subject is affected, preferably is diagnosed, with a bacterial infection. In one embodiment, the subject is at risk of developing a bacterial infection. Examples of risks factor include, but are not limited to, genetic predisposition, or familial history of bacterial diseases, or comorbidities resulting in chronic wounds/ulcers (venous ulcers, pressure ulcers, diabetic foot ulcers, and ischemic ulcers).

### Examples

### Example 1. Synthesis of a magnetic anisotropic material as defined in the first object of the present invention

Maghemite nanocrystals were synthesized using a precipitation method from an aqueous solution of iron sulphates. In brief, a solution of Fe²⁺ (0.027 mol/l) and Fe³⁺ ions (0.023 mol/l) were precipitated with aqueous ammonia (25% by mass) in two steps. In the first step, the pH value of the solution was raised to pH 3 and maintained at a constant value for 30 min to precipitate the iron hydroxides. In the second step, the pH value was further increased to pH >11 to oxidize the iron (II) hydroxide with oxygen from the air, forming a spinel product. After an ageing time of 30 min, the synthesized particles were thoroughly washed with a diluted ammonia solution at pH 10.5.

Then, approximately 75 maghemite nanocrystals are self-assembled into a nanoparticle cluster according to the following protocol.

The nanoparticle clusters were prepared by an emulsification method. Maghemite nanocrystals were suspended in dichloromethane (ca. 5 vol% of the continuous phase) which corresponds to an inner phase. The inner phase is emulsified with water as a continuous phase by stirring the reaction mixture at 700 rpm. The polyacrylic acid (PAA, Mw = 25 kDa, ~0.1wt.% of continuous phase) and polyvinylpyrrolidone (PVP, Mw = 45 kDa, ~1wt.% of continuous phase) were dispersed in said continuous phase and they help to stabilize the droplets. The residual solvent in droplets was removed using a rotary evaporator.

The nanoparticle clusters are also commercially available from Nanos Scientificae d.o.o. (Nanos SCI) company (Ljubljana, Slovenia) under the trade name iNANOvative.

Subsequently, the nanochains are synthesized by magnetic field-induced assembly of these nanoparticle clusters. The nanochain structure is fixated by silica deposition in the form of a thin shell, which is formed by adjusted Stober method. Silica shell thickness can be easily tuned by the amount of added tetraethoxysilane. More particularly, the protocol to obtain the nanochains is as follows: a typical synthesis for the nanochains formation was carried out at a polyvinylpyrrolidone concentration of 1.25 × 10⁻⁴ M, with exposure to a magnetic field of (5.2 ± 1.2) × 10⁴ A m⁻¹ for 85 min after the addition of tetraethyl orthosilicate, a nanocluster concentration of 1.6 × 10⁻⁸ M, and a tetraethyl orthosilicate concentration of 60 mM. The tetraethyl orthosilicate was added 10 min after the transfer of the nanoclusters into the polyvinylpyrrolidone solution. The pH value was set to 8.5, using aqueous ammonia 0.5% by mass, after 80 min of tetraethyl orthosilicate addition. The nanochain synthesis was completed in 3 h.

The polyvinylpyrrolidone was used as a promoter for further silica-shell synthesis, since it is suitable for the colloidal stability of the system.

Then, carboxylate groups were introduced to the silica-coated nanochains according to a protocol as follows: the nanochain surface was modified by functionalization with carboxyl-carrying moieties. In brief, nanochains (150 mg, 15 mL distilled water) were diluted with 15 mL of ethanol to which 75 µL of aminosilane and 250 µL of NH₄OH were added while the reaction mixture was stirred mechanically with glass propeller at 400 rpm for 5 h at 55 °C. The synthesized nanochains-NH₂ were magnetically separated from the suspension and washed first with ethanol and then rinsed 2 times with distilled water and transferred in dimethylformamide. For the preparation of carboxyl-functionalized nanochains (nanochains-COOH), the nanochains-NH2 were further reacted with succinic anhydride (SA), applying a ring-opening elongation reaction. The nanochain-NH₂ suspension (50 mg in 35 mL dimethylformamide) was led to react with SA (10 mg in 15 mL DMF) solution, where a 0.5 mL aliquot of the solution of SA in dimethylformamide was added per minute into nanochain-NHz suspension, while being stirred mechanically with a glass propeller at 600 rpm overnight at room temperature. The produced nanochains-COOH formed a colloidal aqueous suspension.

These carboxyl-functionalized silica-coated iron oxide nanochains were used in the next experiments.

The superparamagnetic anisotropic material is also commercially available from Nanos SCI company (Ljubljana, Slovenia) under the trade name iNANOvative.

### Magnetic anisotropic material characterization

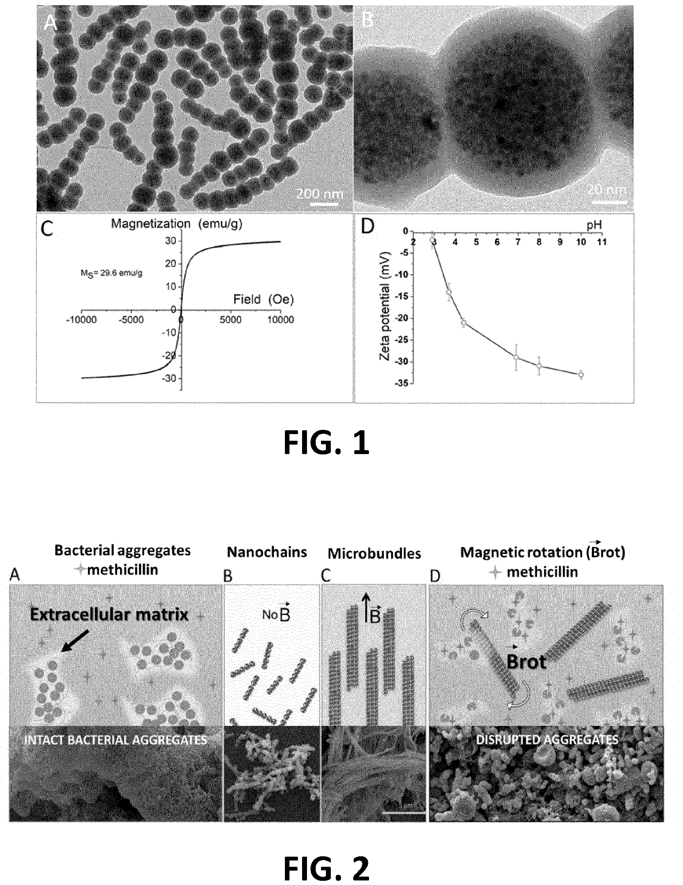

Figure 1 represents the structure and properties of a magnetic anisotropic material as defined in the first object of the present invention: figure 1A) is a transmission electron microscopy (TEM) micrograph of superparamagnetic nanochains showing nanochains general structure, figure 1B) is high resolution TEM micrograph showing electron dense superparamagnetic clusters or cores and less contrasted amorphous silica shell, which makes the nanochains permanently aligned in 1D anisotropic shape, figure 1C) represents room-temperature measurement of the magnetization as a function of the applied magnetic field (Field) which confirms the superparamagnetic nature of the material, and figure 1D) represents the zeta potential curve as a function of pH for superparamagnetic nanochains aqueous suspension.

The magnetic anisotropic material as defined in the first object of the present invention has anisotropic shape and is in the form of magnetic nanochains (~120 nanochains analysed), each nanochain comprising approximately 5 clusters of maghemite (iron oxide) nanocrystals (nanoparticles). The mean nanoparticle cluster size determined from TEM images (150 particles counted) was 110 nm. Each cluster (size ~110 nm) is composed of ~75 superparamagnetic maghemite nanocrystals or nanoparticles (size ~10 nm). The core-shell nature of the nanochains, with the aligned nanocrystal clusters or cores and the amorphous silica shell, can be clearly distinguished in TEM micrographs shown in figure 1 (figures 1A and 1B). The thickness of the silica shell is around 20 nm. The mass of a single nanochain composed of 5 nanoparticle clusters is estimated to be 2.1×10⁻¹⁴ g. Iron oxide represents approximately 45% of the nanochains' mass.

The nanochains are superparamagnetic with saturation magnetization (Ms) of 29.6 emu/g and they exhibit good colloidal stability and high magnetic responsiveness (figure 1C). Therefore, when a suspension of nanochains is exposed to a magnetic field gradient, the nanochains rapidly form transient bundles and drift in the direction of the magnetic field gradient. Subsequently, as soon as this relatively weak magnetic field is removed, the microbundles are spontaneously disassembled back to individual nanochains, due to their colloidal stability and superparamagnetism. Moreover, an exposure of the nanochains suspension to the rotating magnetic field (2 Hz, with the relatively uniform magnetic field of ~2.5 mT), generated by conventional laboratory magnetic stirrer, results in both transient *in situ* microbundles formation and the axial microbundles rotation at a frequency of 2 Hz.

The values of zeta potential of magnetic nanochains were measured as function of the operational pH value (figure 1D). The nanochains showed an acidic character because of the presence of negatively charged form of carboxyl groups at pH values above the isoelectric point, which is at pH ~2.8. The zeta potential of nanochains reached high negative values at a physiological pH of 7.4 at - 31 mV. The high absolute values of the zeta potential provided strong electrostatic repulsive forces between nanochains, resulting in good colloidal stability of the suspension at neutral pH conditions.

### Example 2. Evaluation of the minimal inhibitory concentration (MIC) of methicillin on two bacterial strains

Two strains of *Staphylococcus epidermidis* were used in this study: *S*. *epidermidis* RP62A (ATCC 35984), which is resistant to methicillin and which forms the biofilms, and S. *epidermidis* ATCC 12228, which is sensitive to methicillin and does not form biofilms. A single colony of *S*. *epidermidis* ATCC 35984 or ATCC 12228 was pre-cultivated in Lysogeny broth (LB) medium (Sigma-Aldrich, France) at 37°C under agitation at 200 rpm. After overnight incubation, bacterial growth was monitored by optical density (OD) measurements at 600 nm.

At first, we determined the methicillin inactivation rate or minimum inhibitory concentration (MIC) of the two mentioned strains. A stock solution of 100 mg/mL methicillin (methicillin sodium salt, Sigma-Aldrich, France) was prepared in sterile water, and bacterial strains are put into contact with different concentrations of methicillin to determine the methicillin inactivation rate. Bacteria were inoculated into 200 µL of liquid growth medium with increasing concentrations of methicillin, ranging from 0.3 µg/mL to 3000 µg/mL. The initial OD was 0.1 and the growth was assessed using a CLARIOstar multi-well plate reader, by consecutive OD measurements at 600 nm made in 5 minutes interval over a time range of 20 hours.

The minimal inhibitory concentration (MIC) of methicillin was assayed in lysogeny broth (LB), used to obtain planktonic aggregates in 96-well microtiter plates, and corresponded to the lowest concentration of methicillin that totally inhibited the visible growth of bacteria, as reflected by a loss of increase of the OD. A MIC of 1000 µg/mL was obtained for the resistant strain, ATCC 35984, and a MIC of 3 µg/mL was obtained for the methicillin sensitive strain, ATCC 12228.

Consequently, a sub-inhibitory dose of 300 µg/mL (i.e. 3.3 times lower than the dose that was required to inhibit the growth of the methicillin-resistant strain) was chosen for further experiments in example 3 with *S*. *epidermidis* ATCC 35984 and a sub-inhibitory dose of 0.3 µg/mL (i.e. 10 times lower than the dose that was required to inhibit the growth of the methicillin-sensitive strain) was chosen for further experiments in example 3 with *S*. *epidermidis* ATCC 12228, to assess if the magnetic anisotropic material amplifies the response to the antibiotic.

### Example 3. The magnetic anisotropic material for use in the disruption of bacterial biofilm

Bacterial suspensions are divided in 4 groups as follows:
- *S*. *epidermidis* ATCC 35984: G1 (with the superparamagnetic anisotropic material - iron concentration of 1 mM, no methicillin, magnetic stirring at 120 rpm), G2 (with the superparamagnetic anisotropic material - iron concentration of 1 mM + 300 µg/mL of methicillin, magnetic stirring at 120 rpm);
- *S*. *epidermidis* ATCC 12228: G1' (with the superparamagnetic anisotropic material - iron concentration of 1 mM, no methicillin, magnetic stirring at 120 rpm), G2' (with the superparamagnetic anisotropic material - iron concentration of 1 mM + 0.3 µg/mL of methicillin, magnetic stirring at 120 rpm);
- *S*. *epidermidis* ATCC 35984: GC1 (without the superparamagnetic anisotropic material, no methicillin, mechanical stirring at 120 rpm), GC2 (without the superparamagnetic anisotropic material + 300 µg/mL of methicillin, mechanical stirring at 120 rpm);
- *S*. *epidermidis* ATCC 12228: GC1' (without the superparamagnetic anisotropic material, no methicillin, mechanical stirring at 120 rpm), GC2' (without the superparamagnetic anisotropic material + 0.3 µg/mL of methicillin, mechanical stirring at 120 rpm).

Groups were exposed to specified treatments for 24 hours, following which the bacteria were transferred to agar plates. The colonies were counted 24h after seeding.

The superparamagnetic anisotropic material was applied to bacteria at an iron concentration of 1 mM, which corresponds to approximately 8.55×10⁹ nanochains per milliliter.

Groups G2 and G2' are part of the invention.

Groups G1, G1', GC1, GC2, GC1', GC2' are not part of the invention.

In particular, GC1, GC2, GC1', GC2' are control groups. As magnetic stirring results in the generation of liquid flow movement around bacterial aggregates, mechanical stirring was applied at the approximately same speed of magnetic stirring (120 rpm), with and without methicillin, to reproduce the movement of the local liquid in the control group. Agitation in control groups GC1, GC2, GC1', GC2' not containing the superparamagnetic anisotropic material represents a comparative perturbance factor with respect to groups G1, G1', G2, G2' containing the superparamagnetic anisotropic material. In other terms, mechanical stirring was used in control groups GC1, GC2, GC1', GC2' as a surrogate for magnetic stirring.

It is also noted that magnetic stirring in the absence of a magnetic material does not induce any liquid movement.

### Statistical analysis

Data were collected from investigations made in triplicates performed in three independent experiments. The results are expressed as mean ± SD and the differences between groups were assessed by unpaired Student *t*-test. A *p* value < 0.05 was considered significant.

### Low frequency rotating magnetic field

The low frequency rotating magnetic field was generated with the magnetic stirrer Heidolph Instruments MR Hei-Standard, Germany, at a stirring speed of approximately 120 rpm. The magnetic stirrer consists of a single rod magnet, placed a few centimeters below the surface of the stirrer, which rotates around its short axis. Sample-containing tubes were placed at a distance of approximately 5 cm from the surface of the stirrer, and were thus exposed to a rotating magnetic field with a magnitude between 2.0-2.5 mT, as measured by a Hall magnetometer.

Figure 2 is a schematic representation of figure 2A) cellular aggregates of methicillin-resistant biofilm-forming *Staphylococcus epidermidis*: methicillin is added to these aggregates but no bactericidal effect is observed, of figure 2B) magnetic nanochains homogenously suspended and randomly oriented in the absence of magnetic field, of figure 2C) nanochains forming transient microbundles *in situ* when they are exposed to a magnetic field B, and of figure 2D) microbundles starting to rotate upon submission to a low frequency rotating magnetic field: this rotation in the presence of methicillin resulted in bacterial aggregates size reduction and increased bactericidal effect.

The magnetic anisotropic material of the present invention can thus locally exert physical actions, such as induce mechanical forces following rotational or oscillatory movement, to loosen or disrupt the biofilm upon the application of external stimuli, and deliver antibiotic agents.

### Evaluation of methicillin response in treatment with the superparamagnetic anisotropic material

As described above, different treatments were tested involving the contacting of the superparamagnetic anisotropic material, methicillin as an antibiotic, or a mixture of the superparamagnetic anisotropic material and methicillin, under magnetic or mechanical stirring, with biofilm forming bacteria resistant to methicillin (ATCC 35984) and with biofilm-free (i.e. not forming biofilms) methicillin sensitive bacteria (ATCC 12228).

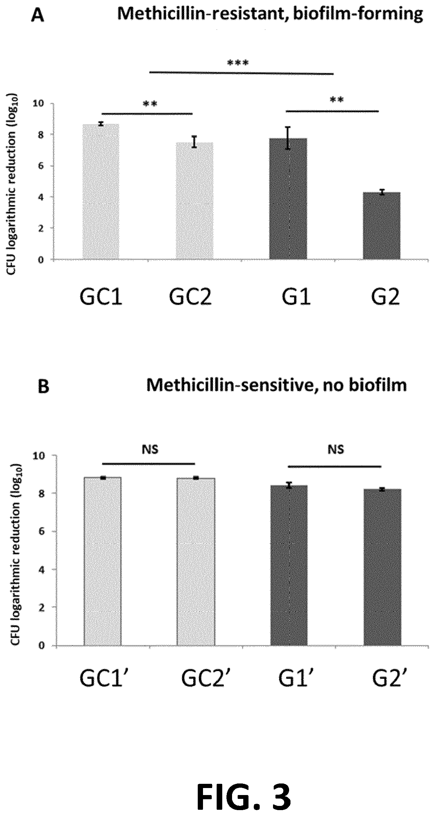

Figure 3 represents mean log₁₀ colony forming units (CFU) reduction obtained for A) *S*. *epidermidis* ATCC 35984 submitted to different treatments corresponding to previously described groups: GC1, GC2, G1 and G2 (figure 3A); or for B) *S*. *epidermidis* ATCC 12228 submitted to different treatments corresponding to previously described groups: GC1', GC2', G1' and G2' (figure 3B).

Figure 3 shows that methicillin only very slightly reduces bacterial growth, despite the mechanical stirring. The superparamagnetic anisotropic material, submitted to magnetic stirring decreases the growth of biofilm-forming bacteria in presence of the antibiotic (figure 3A), but does not alter the growth of bacteria that do not form biofilms (figure 3B).

As a result, in the absence of a biofilm-forming bacteria, the superparamagnetic anisotropic material did not improve antibiotic response, and no reduction of bacterial growth is observed (figure 3B). In the presence of a biofilm-forming bacteria, a log 4 CFU reduction was observed when bacteria were concomitantly exposed to the antibiotic and the superparamagnetic anisotropic material magnetically stirred, showing that magnetically stirring superparamagnetic anisotropic material in the presence of methicillin clearly improved the antibiotic response in biofilm forming bacteria.

Consequently, in the occurrence of biofilm, the method of the present invention dramatically improves the antibiotic sensitivity of biofilm-forming bacteria.

The superparamagnetic anisotropic material can thus loosen or disrupt the bacterial extracellular matrix and improve methicillin penetration/diffusion into bacterial aggregates, to improve methicillin response of biofilm-forming bacteria (figure 3A).

### Electron microscopy

The visualization of bacteria and the extracellular matrix was made using transmission electron microscopy TEM with a Hitachi HT7700 (Tokyo, Japan) transmission electron microscope operating at 80 kV and equipped with a CCD AMT XR41 camera; and scanning electron microscopy- SEM (with a FEI Quanta 250 FEG scanning electron microscope, Thermo Fisher, Hillsboro, OR, USA). Prior TEM observations, bacterial aggregates were fixed (2% glutaraldehyde in 0.1 M sodium cacodylate buffer), post-fixed (1% osmium tetroxide), gradually dehydrated in ethanol, embedded (Embed 812 resin, Electron Microscopy Sciences) and sliced to 70 nm thick sections. Prior SEM the bacteria underwent fixing (2% glutaraldehyde in 0.1 M sodium cacodylate buffer), gradual dehydration in ethanol, critical-point drying, mounting on SEM stubs and platinum sputter coating (Leica EM MED 020). The images were acquired at an accelerating voltage of 5 kV.

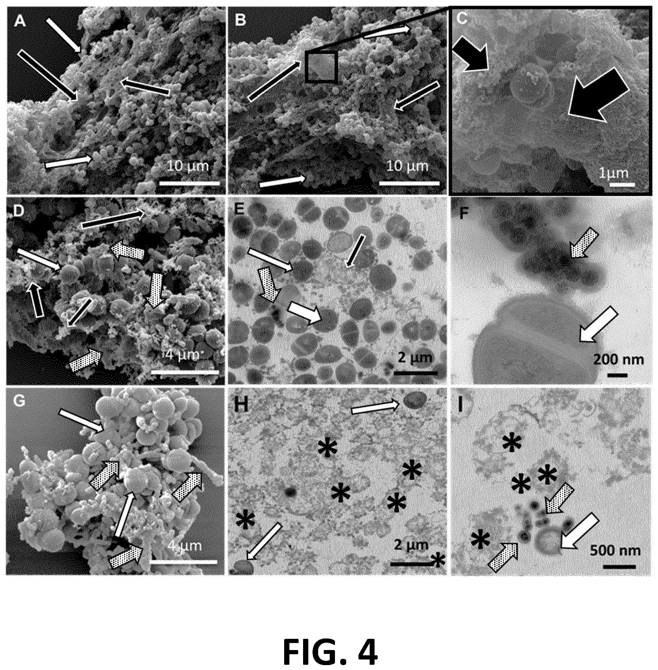

Figure 4 represents electron microscopy images showing representative S. *epidermidis* (ATCC 35984) bacterial aggregates: scanning electron microscopy images of A) group GC1 (i.e. not exposed to methicillin and not exposed to the superparamagnetic anisotropic material with mechanical stirring) (figure 4A), B) group GC2 (i.e. exposed to methicillin and not exposed to the superparamagnetic anisotropic material with mechanical stirring) (figure 4B), C) magnified view of the group GC2, D) group G1 (i.e. exposed to the superparamagnetic anisotropic material without methicillin with magnetic stirring), with corresponding transmission electron microscopy images E and F, showing viable electron dense bacteria secreting the extracellular matrix (figures 4D, 4E and 4F), G) group G2 (i.e. exposed to the superparamagnetic anisotropic material and methicillin with magnetic stirring), with corresponding transmission electron microscopy images H and I, evidencing unviable bacterial debris (figures 4G, 4H and 4I).

White arrows indicate bacteria, black arrows point to the extracellular matrix secreted by bacteria and dotted arrows indicate the superparamagnetic anisotropic material and their bundles, namely microbundles. Bacterial debris are indicated with a black asterisk.

Figure 4 shows the local effect of the superparamagnetic anisotropic material on the extracellular matrix, secreted by the biofilm-forming bacteria (ATCC 35984). As expected, mechanically stirred bacteria form a dense extracellular matrix in the absence of methicillin and in the absence of nanochains (**figure 4A**). The structure of the extracellular matrix is also abundant in the group of bacteria mechanically stirred in the absence of the superparamagnetic anisotropic material and the presence of 300 µg/mL methicillin (**figures 4B and 4C**), and the extracellular matrix is not affected, when the superparamagnetic anisotropic material is added alone, in the absence of methicillin (**figures 4D and 4E**). In the absence of methicillin, the superparamagnetic anisotropic material clearly does not affect the bacterial structure as shown in **figure 4F** since a characteristic electron-dense dividing bacteria coccus, lies close to a small nanochains aggregate. Yet and remarkably, when the superparamagnetic anisotropic material is magnetically stirred with bacteria in the presence of 300 µg/mL methicillin, the extracellular matrix occurrence is drastically reduced (**figure 4G**) and numerous bacterial debris are evidenced (**figures 4H and 4I**). This bactericidal effect is thus noted in presence of the superparamagnetic anisotropic material and the antibiotic with magnetically stirred bacteria.

## Claims

1. A magnetic anisotropic material in the form of magnetic elongated elements, preferably superparamagnetic nanochains, each elongated element comprising core(s) of metal and/or metal oxide particle(s), for use in the disruption of a bacterial biofilm and/or in the sensitization of bacterial colonies to antibiotic treatment.

2. A magnetic anisotropic material for use according to claim 1, wherein each elongated element is encapsulated in an inorganic or organic material shell or is randomly embedded in an inorganic or organic material matrix.

3. A magnetic anisotropic material for use according to claim 2, wherein the inorganic or organic material is selected from metal oxides such as TiOz, Al₂O₃, ZnO, SiOz, polymers, carbon, and silicon, and preferably from metal oxides, such as SiOz.

4. A magnetic anisotropic material for use according to any one of claims 1 to 3, wherein the metal and/or metal oxide particles are selected from nanoparticles comprising at least one metal selected from Fe, Co, and Ni, and preferably the particles are iron oxide nanoparticles.

5. A magnetic anisotropic material for use according to any one of claims 1 to 4, wherein the elongated elements are negatively charged.

6. A magnetic anisotropic material for use according to any one of claims 1 to 5, wherein the cores are aligned so as to form said each elongated element.

7. A magnetic anisotropic material for use according to any one of claims 2 to 6, wherein the shell is an inorganic porous shell.

8. A magnetic anisotropic material for use according to any one of claims 1 to 7, wherein the elongated elements have a shape factor of at least 1.5, and preferably ranging from 3 to 15.

9. Non-therapeutical use of a magnetic anisotropic material as defined in any one of claims 1 to 8, for disrupting a bacterial biofilm and/or sensitizing bacterial colonies present on a surface, on and/or within a medical device, in pipes, in conducts, and/or in a liquid.

10. A biofilm disrupting and/or sensitizing composition comprising a magnetic anisotropic material as defined in any one of claims 1 to 8 and at least one antibacterial agent, preferably selected from β-lactams, aminoglycosides, macrolides, fluoroquinolones, tetracyclines and sulphonamides.

11. A biofilm disrupting and/or sensitizing composition as defined in claim 10, for use in the treatment of a bacterial infection or infestation involving biofilm formation.

12. Non-therapeutic use of a biofilm disrupting and/or sensitizing composition as defined in claim 10, as an anti-infective agent, preferably for the disinfection of a surface, a medical device, pipes, and/or conducts, or for the purification of a liquid.

13. *In vitro* or *ex vivo* method for disrupting a bacterial biofilm and/or sensitizing bacterial colonies to antibiotic treatment, wherein it comprises at least the following steps:
i) contacting a magnetic anisotropic material and an antibacterial agent with a bacterial biofilm or bacterial colonies, and
ii) applying a non-stationary magnetic field, and preferably an oscillating or rotating magnetic field.

14. The method according to claim 13, wherein step i) is implemented by contacting a suspension comprising from 0.05 to 20 mM of metal of said magnetic anisotropic material.

15. The method according to claim 13 or 14, wherein the antibacterial agent is used in a concentration Caa (in µM), where Caa ≤ MICaa/2, and MICaa represents the minimum inhibitory concentration (in µM) of said antibacterial agent with respect to a given bacterial strain.
